# EUROPÄISCHE PATENTSCHRIFT

(11) **EP 0 692 491 B1**
(45) Veröffentlichungstag und Bekanntmachung des Hinweises auf die Patenterteilung: **10.10.2001**
(21) Anmeldenummer: 95110512.1
(22) Anmeldetag: 05.07.1995
(51) Int. Cl.: C07K 16/06, A61K 39/395, C07K 1/14, A61L 2/00

(54) **Virussicheres stabiles pharmazeutisches Präparat bestehend aus wenigstens 70%, bevorzugt 90% humanem monomerem Immunglobulin A und Verfahren zu seiner Herstellung**
Virus safe stable pharmaceutical preparation consisting of at least 70%, preferably 90% human monomeric immunoglobulin A and method for its production
Préparation pharmaceutique stable ne contenant pas d'activité virale consistant d'au moins 70%, préférablement 90% d'immunoglobuline A humaine monomère, et procédé de préparation

(30) Priorität: 14.07.1994 DE 4424935
(43) Veröffentlichungstag der Anmeldung: 17.01.1996
(73) Patentinhaber: Baxter Aktiengesellschaft, 1221 Wien (AT)
(72) Erfinder: Mannhalter, Josef W., Prof. Dr., A-1050 Wien (AT); Leibl, Heinz, Dr., A-1120 Wien (AT); Eibl, Martha, Prof. Dr., A-1180 Wien (AT); Tomasits, Regine, Dipl.-Ing., A-1020 Wien (AT); Wolf, Hermann, Dr., A-1180 Wien (AT)
(74) Vertreter: Kolb, Helga, Dr. Dipl.-Chem.

(56) Entgegenhaltungen:
- EP-A- 0 506 651
- EP-A- 0 525 502
- US-A- 5 258 177
- PROTEIN EXPRESSION AND PURIFICATION, Bd. 6, Nr. 4, August 1995 DULUTH, MN, USA, Seiten 408-410, H. LEIBL ET AL. 'Isolation of human serum IgA using thiophilic adsorption chromatography.'

## Beschreibung

Die Erfindung betrifft ein virussicherem stabiles pharmazeutisches Präparat bestehend aus wenigstens 70%, bevorzugt 90% humanem monomerem Immunglobulin A (IgA) und ein Verfahren zu seiner Herstellung.

Immunglobuline (Ig) sind spezifische Abwehrproteine im Blutplasma, der Lymphe und in anderen Körpersekreten aller Wirbeltiere. Die Immunglobuline werden von B-Lymphocyten synthetisiert. Die monomeren Immunglobuline bestehen aus je zwei L (light) und H (heavy)-Ketten, die durch Disulfid-Brücken miteinander verbunden sind. Die Immunglobuline sind Glycoproteine, die als Antikörper fungieren, und deren Bildung durch Antigene induziert wird. Mengenmässig machen sie ca. 20 % des gesamten Plasma-Proteins aus. Von den Immunglobulinen wurden beim Menschen bisher 5 Hauptklassen identifiziert (IgA, IgD, IgE, IgG und IgM), die sich in ihren H-Ketten, in ihrer Serum-Konzentration, dem Molekulargewicht (ca. 146000 bis 970000), dem Kohlenhydrat-Gehalt, der elektrophoretischen Beweglichkeit und in ihren biologischen Eigenschaften unterscheiden. Die Hauptklassen IgA und IgG lassen sich noch in Subklassen unterteilen (z.B. IgA1, IgA2). Die Vielfalt der Immunglobulin-Klassen und -Unterklassen sowie deren vielfältige, unterschiedliche Spezifität in der Bindung von Antigenen kommt durch Kombination verschiedener vorhandener genetischer Bauteile zustande. Auf der induzierten Bildung von Immunglobulinen beruht die aktive Immunisierung, während bei der passiven Immunisierung gegen verschiedene Virus- und Bakterien infektionen vorab gebildete Immunglobuline zugeführt werden.

Immunglobulin A (IgA) stellt die Hauptantikörperklasse in äusseren Sekreten wie Speichel, Tränenflüssigkeit und dem Schleim des Bronchial- und des Intestinaltraktes dar. Somit dient Immunglobulin A als eine der ersten verteidigungslinien gegen bakterielle und virale Antigene.

Zur Prophylaxe und Behandlung bakterieller und viraler Infektionen wurden bereits pharmazeutische Zusammensetzungen auf der Basis von Immunglobulinen, z.B. von Immunglobulin A, vorgeschlagen und verwendet (vgl. z.B. die JP-A-57-59815).

IgA kann von einem Rezeptor, der sekretorischen Komponente, durch die Epithelzellen hindurch von der Blutseite zur extrazellulären Seite transportiert werden.

In der reinen monomeren Form besteht IgA aus zwei leichten (L) und zwei schweren (H) Ketten; in der dimeren sekretorischen Form sind zwei solche Monomere durch die sogenannte J-Kette (joining chain) zusammengekoppelt. In den Sekreten der Schleimhäute und Drüsen kommen vor allem Dimere mit einer zusätzlichen sekretorischen Komponente (sogenannte SC-Komponente) vor.

In Lösung liegt IgA als IgA-Monomer im Gleichgewicht mit IgA-Dimer vor. In diesem Gleichgewicht beträgt der Anteil an dimerem IgA maximal etwa 25 % des Gesamt-IgA.

IgA besteht aus zwei Subklassen, IgA1 und IgA2, die in einem nativen Verhältnis von etwa 80 Gew.-% zu 20 Gew.-% vorliegen.

Dieses Verhältnis kann im Rahmen einer Isolierung verändert werden. Das native Verhältnis von kappa-zu lambda-Leichtketten in einem Immunglobulinpräparat beträgt etwa 1:1.

IgA stellt nur ca. 3 - 4 % des Gesamtproteins von normalem humanem Serum dar. Da IgA eine ausgeprägte Tendenz zur Komplexbildung und Aggregation während der Reinigung besitzt, ist die Isolierung des monomeren IgA aus Serum meist mit geringen Ausbeute verbundene. Unter den zahlreichen Herstellungsverfahren sind bislang nur wenige bekannt, die auch für eine grosstechnische Produktion geeignet sind.

Hauptverunreinigungen der IgA-Präparationen sind die verschiedenen Unterklassen von Immunglobulin G, deren Abtrennung zusätzliche, die Ausbeute an IgA weiter vermindernde, Reinigungsmethoden erfordern.

Die bekannten und gängigen Methoden zur Reinigung von Immunglobulinen beruhen zumeist auf Unterschieden in den physikalischen Eigenschaften, wie z.B. Löslichkeit in wässerigen Systemen (Reinigung durch fraktionierte Fällung), Anteil an Ladungen (Reinigung durch Ionenaustauschchromatographie), oder Unterschieden in der Molekülgrösse (Reinigung durch Molekularausschlusschromatographie).

In der JP-A-57-59815 wird eine Reinigung von Immunglobulinen aus humanem Ausgangsmaterial beschrieben. Dabei wird nach vorausgehender Fällung mit Ammoniumsulfat eine Gelfiltration an Sephacryl® S-200 und anschliessend zur Abtrennung des IgG von IgA eine Affinitätschromatographie durchgeführt. Aufgrund der sehr ähnlichen Molekulargewichte von monomerem IgA und IgG (IgA:162 kD, IgG:153 kD; vgl. J.F. Heremans, Immunglobulin A, in: The Antigens, Vol. 2 [1974], Seite 365-522; Academic Press New York) ist eine Isolierung von monomerem IgA allerdings schwierig. Obwohl das gereinigte IgA als aktiver Bestandteil in pharmazeutischen Zusammensetzungen verwendet wird, und zur Reinigung humanes Ausgangsmaterial eingesetzt wird, werden gemäss JP-A-57-59815 keine Massnahmen zur Inaktivierung von Viren beschrieben. Zudem liegt das IgA in Kapseln oder Pellets vor, die keinem Verfahren zur Inaktivierung von Viren unterzogen werden können und im speziellen nicht hitzebehandelt werden können.

A. Collard et al., Ann. Rech. Vét. 15 (4) (1984) 497-501 beschreiben eine Reinigung der Immunglobuline A, G und M aus Rinderblutserum oder aus Colostrum mittels Gelfiltration an Sephacryl® S-300. Durch dieses Verfahren kann IgA auch aus Blutserum erhalten werden. Die Ausbeute ist aber gering und das Endprodukt ist überdies mit IgG1- und IgG2-Subklassen verunreinigt; ausserdem gelingt eine Trennung zwischen IgA und IgG aus Serumproben schlechter als aus Colostrum. In Colostrum allerdings liegt IgA primär als sekretorisches IgA vor, welches aus einem Komplex von IgA-Dimeren und zusätzlichen Proteinketten besteht.

Nach J.R. Patterson et al., J. clin. Path. 28 (1975) 670-673, werden zwei unterschiedliche Gelfiltrationsmedien, nämlich Sephadex® G-200 und Bio-Gel® A-5M, zur Trennung von Immunglobulinen aus Seren verwendet. Bei der so erhaltenen IgA-Fraktion handelt es sich aber nicht um eine monomere IgA-Präparation, sondern um eine IgA-Präparation mit hohem Molekulargewicht.

Ein Problem bei Verwendung von humanem Ausgangsmaterial für die Herstellung von Immunglobulinen ist jedoch die Virussicherheit des erhaltenen Produktes. Trotz Spenderauswahl und Testung der Einzelspenderplasmen ist nicht auszuschliessen, dass z.B. aufgrund der geringen Empfindlichkeit mancher Tests noch infektiöse Krankheitserreger, insbesondere Hepatitisviren oder Retroviren, wie HIV, im Spenderpool vorhanden sind.

Obwohl bei der Herstellung einer Immunglobulinpräparation durch fraktionierte Alkoholfällung nach Cohn eine Abreicherung/Inaktivierung von Viren um mehr als 10¹⁵ Einheiten erfolgt (vgl. z.B. Wells et al., Transfusion 26 (1986) 210-213), besteht, insbesondere bei Anwendung eines neuen Reinigungsverfahrens, immer noch die Gefahr einer unzureichenden Vireninaktivierung, d.h. einer nicht ausreichenden Virussicherheit.

Um eine ausreichende Inaktivierung von Viren zu erreichen, kann beispielsweise eine Hitzebehandlung durchgeführt werden. Gegenüber anderen Verfahren zur Inaktivierung von Viren, wie beispielsweise einer Behandlung mit einem Solvens/Detergens-System nach EP-B-0131740, hat die Hitzebehandlung den Vorteil, dass auch nicht-lipidumhüllte Viren, wie beispielsweise Hepatitis A Viren, inaktiviert werden.

Ein Nachteil der Hitzebehandlung, aber auch anderer Verfahren zur Inaktivierung von Viren, ist es jedoch, dass ein wesentlicher Anteil an IgA multimerisiert bzw. polymerisiert werden kann.

Bei gängigen Verfahren zur Inaktivierung von Viren und speziell bei der Hitzebehandlung muss mit einer Aggregatbildung der Immunglobuline gerechnet werden. Derartige Aggregate verursachen aber u.a. eine Erhöhung der antikomplementären Aktivität und damit Unverträglichkeitsreaktionen nach intravenöser Verabreichung. Deshalb wird bei einigen Reinigungsverfahren, in denen ein Schritt zur Inaktivierung der Viren oder im speziellen eine Hitzebehandlung durchgeführt wird, in Gegenwart von Stabilisatoren gearbeitet.

Eine Hitzebehandlung in Gegenwart von Stabilisatoren wird z.B. in der EP-B-0177836 beschrieben; danach wird ein lyophilisiertes Immunglobulin-G mit einem Feuchtigkeitsgehalt von 3 % oder weniger unter Zusatz eines Stabilisators 10 Minuten bis 200 Stunden lang auf 30° C bis 100 °C erhitzt. Der Stabilisator schützt die relativ labilen Immunglobuline vor Denaturierung und erhält somit deren biologische Aktivität.

Ein Nachteil der Hitzebehandlung in Gegenwart von Stabilisatoren ist es aber, dass diese Stabilisatoren dann auch im Endprodukt enthalten sind und deshalb anschliessend abgetrennt werden müssen.

Eine Hitzebehandlung ohne Stabilisatoren wird z.B. in der EP-B-0159311 beschrieben. Danach werden die Blutprodukte in feuchtem (zwischen 5 und 70 % Wassergehalt) festen Zustand während einer Sekunde bis 100 Stunden erhitzt. Anstelle von Wasser können auch hydroxylgruppenhaltige Verbindungen, wie z.B. Methanol, Ethanol oder Mannit, verwendet werden.

In dem US-Patent 5,528,177 wird eine Methode zur Herstellung einer IgA angereicherten Präparation beschrieben. Dabei werden aber IgA Antikörper im allgemeinen aufgereinigt und nicht speziell monomere IgA Antikörper. Des Weiteren enthalten alle in diesem Dokument beschriebenen Präparationen mindestens 30 bis 40% IgG.

EP-A-0525502 beschreibt ein Verfahren zur Herstellung von virusinaktivierten Immunglobulinlösungen. Diese Lösungen können intravenös injiziert werden und sind virusfrei.

Aufgabe der vorliegenden Erfindung ist es, ein pharmazeutisches Präparat enthaltend humanes virussicheres monomers Immunglobulin A (IgA) bereitzustellen, das im wesentlichen frei von Immunglobulin G (IgG) ist, und das auf einfache und sichere Weise erhalten werden kann, und mit dem sich die vorstehend im Zusammenhang mit dem Stand der Technik aufgezeigten Probleme vermeiden lassen. Diese Aufgabe wird mit dem Gegenstand der vorliegenden Erfindung gelöst.

Gegenstand der Erfindung ist ein pharmazeutisches Präparat enthaltend virussicheres monomeres IgA gemäss Patentanspruch 1, das im wesentlichen frei ist von IgG und durch Reinigung einer Immunglobulin-haltigen Fraktion und Durchführung eines Verfahrens zur Inaktivierung von Viren erhältlich ist.

"Im wesentlichen frei von IgG" bedeutet, dass der Anteil an IgG am Gesamtimmunglobulin weniger als 10 Gew.-% beträgt, und vorzugsweise weniger als 5 Gew.-%, und insbesondere weniger als 3 Gew.-%.

Vorzugsweise ist das monomere IgA auch im wesentlichen frei von IgG-Dimeren. "Im wesentlichen frei von IgG-Dimeren" bedeutet dabei, dass der Anteil an IgG-Dimeren am Gesamtimmunglobulin kleiner als 5 Gew.-% ist, und vorzugsweise kleiner als 3 Gew.-%, und insbesondere unter der Nachweisgrenze der Radialimmundiffusion (RID) liegt.

Das erfindungsgemäss hergestellte und verwendete humane virussichere monomere IgA ist vorzugsweise auch frei von Fibrinogen und/oder frei von Plasminogen. Frei von Fibrinogen und/oder Plasminogen bedeutet dabei, dass diese Verunreinigungen mit herkömmlichen Tests, wie z.B. mittels Radialimmundiffusion (RID), Gerinnungstests oder Fibrinolysetests, nicht nachweisbar sind. Auf diese Weise wird sichergestellt, dass in den erfindungsgemässen Präparaten und diese enthaltenden pharmazeutischen Zusammensetzungen kein Stoff enthalten ist, der bei der Verabreichung des Immunglobulins den Organismus zusätzlich belasten könnte.

Bevorzugte Ausführungsformen davon sind Gegenstand der Ansprüche 2 bis 7.

Weiterer Gegenstand der Erfindung ist ein Verfahren gemäss Anspruch 8 zur Herstellung eines virussicheren stabilen pharmazeutischen Präparat bestehend aus wenigstens 70%, bevorzugt 90% humanem, monomerem IgA.

Zweckmässige Ausgestaltungen dieses Verfahrens sind Gegenstand der Ansprüche 9 bis 17.

Nach dem erfindungsgemässen Verfahren ist es überraschenderweise möglich, ein IgA, das im wesentlichen frei ist von IgG zu erhalten, das als monomeres IgA vorliegt und als virussicher angesehen werden kann, d.h. die Infektion mit Viren nach Anwendung dieses Produktes kann ausgeschlossen werden.

Unter monomerem IgA wird erfindunsgemäss ein IgA-Präparat verstanden, das mindestens 96 Gew.-% monomeres IgA, bezogen auf die Gesamtmenge an IgA, enthält. Der Anteil an monomerem IgA in dem stabilen pharmazeutischen Präparat beträgt vorzugsweise wenigstens 70%, am meisten bevorzugt 90%. Aufgrund der hohen Tendenz von Immunglobulinen, bei der Behandlung mit chemischen Substanzen, wie sie in Verfahren zur Inaktivierung von Viren verwendet werden oder auch bei Hitzebehandlung, Aggregate zu bilden, bestand gegenüber einer Behandlung zur Inaktivierung von Viren und im speziellen einer Hitzebehandlung bisher eher ein Vorurteil. Überraschenderweise wurde nun gefunden, dass nach dem erfindungsgemässen Verfahren auch bei der Durchführung eines Verfahrens zur Inaktivierung von Viren gemäss Verfahrensstufe (b) das IgA keiner Aggregatbildung unterliegt, auch wenn beispielsweise eine Hitzebehandlung ohne Zusatz üblicher Stabilisatoren, wie z.B. von Kohlenhydraten oder Aminosäuren, durchgeführt wird. Eine bevorzugte Ausführungsform sieht eine Hitzebehandlung vor, und speziell die Hitzebehandlung in Lösung, wobei Polyethylenglykol in einer nicht-präzipitierenden Menge zur Verbesserung der Virusinaktivierenden Wirkung vorliegt. Dabei ist die Verwendung von niedermolekularem PEG bevorzugt.

Das im erfindungsgemässen Verfahren eingesetzte, humanes IgA enthaltende Ausgangsmaterial ist vorzugsweise humanes Blutserum, Plasma und/oder Plasmafraktionen.

Zweckmässigerweise werden das Plasma und/oder die Plasmafraktionen nach der Methode von Cohn mit Ethanol behandelt, und die Cohn II + III-Fraktion wird als Ausgangsmaterial eingesetzt.

Das IgA-enthaltende Ausgangsmaterial wird mittels Gelpermationschromatographie und/oder eine thiophile Chromatographie gereinigt. Das IgA-enthaltende Ausgangsmaterial kann auch vorgereinigt sein, beispielsweise durch eine Fällung mit Ethanol, Polyethylenglykol und/oder Ammoniumsulfat, und/oder durch ein weiteres chromatographisches Verfahren, z.B. unter Einsatz eines Ionenaustauschers, einer Adsorption an Hydroxylapatit, und/oder Dextransulfat und/oder eine Reinigung an Heparin-Sepharose®. Weitere Reinigungsmethoden umfassen eine Fällung verunreinigender Proteine mit ZnSO₄ oder Rivanol. Durch eine solche Vorreinigung können die für die weiteren Schritte benötigten Chromatographiematerialien, beispielsweise das in der Gelpermeationschromatographie verwendete Gel, geschont werden. Dadurch kann eine längere Verwendung der Chromatographiesäule erreicht werden, weshalb sich das erfindungsgemässe Verfahren auch besonders für einen Einsatz im grosstechnischen Massstab eignet.

Zur Abtrennung von IgG-Dimeren wird vorzugsweise zusätzlich eine Affinitätschromatographie durchgeführt, z.B. an immobilisiertem Protein A oder G, oder an anti-IgG Antikörper, welche an eine feste Matrix gekoppelt sind.

Diese zusätzliche Affinitätschromatographie kann vor der Verfahrensstufe (a), zwischen der Verfahrensstufe (a) und (b) und/oder nach der Verfahrensstufe (b) durchgeführt werden;.. zweckmässigerweise wird sie vor oder nach der chromatographischen Reinigung durchgeführt.

Zur Durchführung eines Verfahrens gemäss Verfahrensstufe (b) wird das in den vorausgehenden Stufen erhaltene Produkt zweckmässigerweise in gelöster oder fester Form eingesetzt. Bei einer Hitzebehandlung in fester Form besitzt das der Hitzebehandlung zu unterziehende Produkt vorzugsweise einen Feuchtigkeitsgehalt von 5 bis 70 Gew.-%. In einer anderen bevorzugten Ausführungsform wird als feste Form ein Lyophilisat eingesetzt. Wird gemäss Verfahrensstufe (b) eine Hitzebehandlung durchgeführt, so erfolgt diese vorzugsweise bei einer Temperatur im Bereich zwischen 40 °C und 80 °C und insbesondere im Bereich zwischen 50 °C und 65 °C. Die Hitzebehandlung wird dabei mindestens zu einem für die Inaktivierung von Viren ausreichend langen Zeitraum durchgeführt, und vorzugsweise während einer Dauer von 30 Minuten bis 10 Stunden. Vorzugsweise erfolgt die Hitzebehandlung als Dampfbehandlung, und insbesondere nach einem in der EP-A-0159311 beschriebenen Verfahren.

Ausser einer Hitzebehandlung können aber auch alle anderen Verfahren zur Inaktivierung von Viren verwendet werden. So kann beispielsweise eine Solvent/Detergenz-Behandlung nach EP-B-0-131 740 oder eine Detergenz-Behandlung nach EP-B-0 050 061 angewendet werden. Auch Methoden, die eine UV-Bestrahlung zusammen mit β-Propiolacton als Verfahren zur Inaktivierung von Viren beinhalten, sind geeignet.

Als weitere Verfahren, Immunglobulin-haltige Fraktionen einem Verfahren zur Inaktivierung von infektiösen Agenzien zu unterwerfen, sind chemische oder physikalische Behandlungen in Gegenwart eines Polyethers zielführend, wie dies in DE-44 34 538 beschrieben ist. Als Polyether kommen zu diesem Zwecke Polyhydroxyether, wie beispielsweise Polyalkylenglykol, und insbesondere Polyethylen- oder Polypropylenglykol in Frage.

Als weitere Möglichkeit zur Inaktivierung vermehrungsfähiger Krankheitserreger soll auch die Verwendung neutraler Peptidhydrolasen, wie beispielsweise Trypsin oder Chymotrypsin, gemäss EP-B-0247998, erwähnt werden.

Es kann auch zweckmässig sein, die Hitzebehandlung mit einem oder mehreren anderen zur Vireninaktivierung bekannten und üblichen Verfahren zu kombinieren, und insbesondere mit einer UV-Bestrahlung, Behandlung mit Tensiden, und/oder mit einer Behandlung mit einem Solvens/Detergent-System. Die einzelnen Verfahrensschritte können dabei gleichzeitig (z.B. Hitzebehandlung unter gleichzeitiger UV-Bestrahlung), oder in beliebiger Reihenfolge hintereinander erfolgen.

Durch eine Kombination der Hitzebehandlung mit einem oder mehreren anderen Verfahren zur Virusinaktivierung lassen sich die unterschiedlichen Wirkungsmechanismen, die bei diesen Inaktivierungsverfahren wirken, einsetzen, wodurch die Virussicherheit des Produktes noch weiter erhöht werden kann.

Vor der Hitzebehandlung kann zweckmässigerweise auch eine Dialyse gegen Wasser durchgeführt werden, wodurch sich gegebenenfalls noch vorhandene Verunreinigungen, und insbesondere gegebenenfalls eingesetzte oder vorhandene stabilisierende Substanzen, weitgehend entfernen lassen.

Wird gemäss Verfahrensstufe (a) eine Gelpermeationschromatographie durchgeführt, erfolgt die Auftrennung von Molekülen aufgrund ihrer unterschiedlichen Molekulargrösse, wobei das Gel als Molekularsieb wirkt. Die Durchführung einer Gelpermeationschromatographie ist deshalb normalerweise unabhängig von der angewandten Ionenstärke. Als Materialien für die Gelpermeationschromatographie können für die Gelpermeationschromatographie übliche Materialien eingesetzt werden, z.B. Superose®, Sephacryl®, Sepharose® oder Sephadex®.

Beim erfindungsgemässen Verfahren hat sich nun überraschenderweise gezeigt, dass eine Trennung des IgG von monomerem IgA an einigen zur Gelpermeationschromatographie verwendeten Materialien auch von der Ionenstärke abhängig ist. Daraus ist zu schliessen, dass bei solchen Gelen noch zusätzliche Wechselwirkungen hydrophil/hydrophober und/oder elektrostatischer Art zur Wirkung kommen.

Vorzugsweise wird deshalb erfindungsgemäss eine hydrophil/hydrophobe und/oder elektrostatische Gelpermeationschromatographie durchgeführt, d.h. eine Gelpermeationschromatographie, in der eine Matrix verwendet wird, die gleichzeitig auch eine hydrophil/hydrophobe bzw. elektrostatische Interaktions- und Affinitätswirkung aufweist. Damit lässt sich eine hervorragende Trennung von IgG und IgA erzielen, die überraschenderweise besser ist als unter Verwendung eines vorstehend genannten üblichen Materials für die Gelpermeationschromatographie.

Für die Gelpermeationschromatographie wird erfindungsgemäss vorzugsweise ein Gel aus quervernetzter Agarose, wie es für die Fast Protein Liquid-Gelchromatographie (FPLC®) von Biomolekülen eingesetzt wird, z.B. Superdex® oder Superose® (beide von Pharmacia), oder ein Gel aus einem synthetischen Material verwendet.

Anstelle der Gelpermeationschromatographie kann auch eine thiophile Chromatographie durchgeführt werden, und auch die Kombination beider Reinigungsverfahren ist möglich. Geeignete Adsorbentien für die thiophile Chromatographie sind bevorzugterweise sogenannte T-Gele auf Basis von Kohlenhydraten oder synthetischen Trägermaterialien, welche Strukturen, wie R-SO₂-CH₂- oder R-S-CH₂-CH₂-SO₂- enthalten und gegen schwefelhaltige Strukturen des Proteins gerichtet sind.

Die Adsorption von Plasma oder einer Plasmafraktion an einer thiophilen Matrix und die anschliessende Elution einer Fraktion, enthaltend monomeres IgA, stellt auch ein geeignetes Verfahren zur Herstellung einer monomeres IgA enthaltenden Präparation dar.

Als humanes virussicheres monomeres IgA wird ein solches hergestellt und verwendet, das im wesentlichen frei ist von IgG.

Im erfindungsgemäss hergestellten und verwendeten IgA liegen die Subklassen IgA1 und IgA2 vorzugsweise in einem Verhältnis vor, dass der Zusammensetzung von nativem IgA entspricht.

Gegenstand der Erfindung ist ebenfalls eine pharmazeutische Zubereitung, die ein humanes virussicheres monomeres IgA, gegebenenfalls zusammen mit üblichen pharmazeutischen Träger- und/oder Verdünnungsmitteln enthält.

Durch Verwendung des erfindungsgemäss herstellbaren IgA ist es möglich, eine pharmazeutische Zusammensetzung bereitzustellen, die stabil und virussicher ist, und die auch noch zusätzlich hitzebehandelt werden kann.

Die erfindungsgemässe pharmazeutische Zusammensetzung kann neben dem IgA auch noch andere aktive Bestandteile (Wirksubstanzen) enthalten, sofern sie mit IgA kompatibel sind und für die Zweckbestimmung der pharmazeutischen Zusammensetzung geeignet und zweckmässig sind.

Die erfindungsgemässen pharmazeutischen Zusammensetzungen sind insbesondere zur Vorbeugung und Behandlung von Entzündungen, Infektionen und/oder Allergien geeignet.

Als pharmazeutische Träger- und Verdünnungsmittel können hierfür übliche pharmazeutisch annehmbare Träger- und Verdünnungsmittel verwendet werden.

Die Herstellung der pharmazeutischen Zusammensetzungen erfolgt nach bekannten und an sich üblichen Verfahren, und richtet sich insbesondere nach der Art der beabsichtigten Darreichungsform.

Die Verabreichung von IgA kann auf lokalem, mukosalem, z.B. oralem oder systemischem Weg durchgeführt werden.

Die Dosierungen hängen vom Verabreichungsweg und der Häufigkeit der Verabreichung ab, sowie vom Ausmass und der Schwere der Erkrankung, z.B. Entzündung. Wenn hohe Gesamtdosen an IgA verabreicht werden, ist es oft bevorzugt, das IgA in mehreren kleineren Dosierungsmengen während des Tages verteilt zu verabreichen. Diese Überlegungen im Hinblick auf die Dosierung und den Verabreichungsweg sind einem Fachmann auf diesem Gebiet allgemein bekannt.

IgA kann z.B. oral (normalerweise 1 bis 10 g/Tag oder, in schweren Fällen, mehr) vorzugsweise in 3 oder mehr Dosen erfolgen.

Immunglobulin kann auch mukosal verabreicht werden, z.B. mittels Inhalationen (bis zu 10 ml/Tag, 10 bis 100 mg IgA/ml), oder nasal (15 bis 200 mg/ml) mittels Sprays oder Tropfen, oder lokal durch intraartikulare Injektionen (die nach Bedarf 1 bis 5 ml einer Lösung von 10 bis 100 mg IgA/ml enthalten). Andere Verabreichungswege umfassen Suppositorien (100 bis 1000 mg/IgA/Dosis) und transdermale Pflaster. Transdermale Pflaster können verwendet werden, um Hautentzündungen zu behandeln.

Als Darreichungsformen kommen insbesondere solche in Frage, die für die Vorbeugung und Behandlung von Virusinfektionen üblich sind, und insbesondere orale Darreichungsformen, wie z.B. Kapseln, Tabletten, Granulate, Pellets, Mini-Pellets, Mikrokapseln, die zur Behandlung von viralen Darminfektionen vorzugsweise mit einem dafür üblichen magensaftresistenten, darmlöslichen Überzug versehen sind.

Für eine prophylaktische und therapeutische Behandlung beim Menschen beträgt die tägliche Verabreichungsdosis der erfindungsgemässen pharmazeutischen Zusammensetzungen in der Regel 10 bis 5000 mg, bezogen auf das erfindungsgemässe monomere IgA. Sie richtet sich aber insbesondere auch nach dem Allgemeinzustand und Alter des Patienten, und nach der Schwere der Erkrankung.

Die nachfolgenden Beispiele sollen nun die Erfindung näher erläutern, ohne sie darauf zu beschränken.

Nachfolgend sind die den Beispielen zugeordneten Abbildungen und ihre Bedeutung angegeben:
Abb. 1
   Bsp. 1
   Säule: Superde® 200 HR 35/600
   Präparativer Lauf zur Isolierung von IgA aus Cohn III NS
Abb. 2
   Bsp. 1
   Säule: Superdex®200 HR 10/30
   Analyse des Endproduktes (IgA)
Abb. 3
   Bsp. 2
   IgA: Gelelektrophorese/ Densitometrie
Abb. 4
   Bsp. 3
   IgA: Gelelektrophorese/ Densitometrie
Abb. 5
   Bsp. 4
   IgA: Gelelektrophorese/ Densitometrie
Abb. 6
   Bsp. 4
   Säule: Superdex® 200 HR 10/30
   IgA Endprodukt ("M" Monomere, "D" Dimere, "A" Aggregate)
Abb. 7
   Bsp. 6
   E.Coli beladen mit IgA
   X-Achse: Intensität der Fluoreszenz
   Y-Achse: Anzahl der detektierten Bakterien
Abb. 8
   Bsp. 7
   Säule: Superdex® 200 HR 10/30
   IgA nach Lyophilisation in PBS rekonstituiert
Abb. 9
   Bsp. 8
   Säule: Superdex 200® HR 10/30
   IgA nach Hitzebehandlung (60 °C, 30 min)
Abb. 10
   Bsp. 8
   Säule: Superdex 200® HR 10/30
   IgA nach Hitzebehandlung (40 °C, 8 Std.)
Abb. 11
   Bsp. 8
   Säule: Superdex 200® HR 10/30
   IgA nach Hitzebehandlung (40 °C, 8 Std. + 50 °C, 8 Std.)
Abb. 12
   Bsp. 9
   Säule: Superdex 200® HR 10/30
   IgA und IgG Auftrennung mit Laufpuffer: PBS (0,15 M NaCl)
Abb. 13
   Bsp. 9
   Säule: Superose® 6 HR 10/30
   IgA und IgG Auftrennung mit Laufpuffer: PBS (0,15 M NaCl)
Abb. 14
   Bsp. 9
   Säule: Superdex® 200 HR 10/30
   IgA und IgG Auftrennnung mit Laufpuffer: PBS ad 2 M NaCl

Bei allen Säulenläufen stellt die x-Achse das Elutionsvolumen in ml dar.

Es wurden die folgenden Abkürzungen verwendet:
OD ... optische Dichte
Hp ... Haptoglobin-Untereinheiten
LC ... leichte Kette
HC ... schwere Kette
M ... Monomere
D ... Dimere
A ... Aggregate
PBS .. Phosphat buffered saline

### BEISPIEL 1

Wie in der Patentanmeldung EP 0506 651 beschrieben, wird aus humanem Plasma eine Cohn II+III-Fraktion hergestellt, die mit einem Phosphat-Acetat-Puffer extrahiert wird. Dieses Material wird mit Äthanol bei einem pH-Wert von 5,3 und bei -2°C auf eine Konzentration von 12 % versetzt, wobei sich ein Niederschlag bildet, der abgetrennt und bei -20°C gelagert wird. Diese bei -20°C gelagerte Paste wird nun folgendermassen behandelt: Pro Gramm Paste werden 25 ml einer 0,9 % NaCl Lösung, die 10 mg Sojabohnen Trypsin Inhibitor (Sigma, St. Louis, MO, USA; Typ I-S) pro ml enthält, zugesetzt. Die Paste wird durch Rühren bei 4°C über Nacht in Suspension gebracht, wobei ein Teil der Proteine gelöst wird. Die Suspension wird 60 min mit 18900 x g bei 4°C zur Abtrennung der unlöslichen Bestandteile abzentrifugiert. Der Niederschlag wird verworfen. Der Überstand wird mit Heparin-Sepharose CL-6B (Pharmacia-LKB, Uppsala, Schweden) versetzt und über Nacht bei 4°C gerührt. Dann wird das Gel über ein Nylonfilter (85 mm Maschenweite) vom Überstand getrennt. Das Gel wird 2 x mit 0,9 % NaCl Lösung gewaschen (jeweils mit 1/4 des Probenvolumen). Überstand und Waschflüssigkeiten werden vereinigt.

Das von der Heparin Sepharose® CL-6B nicht gebundene Material wird mit einer Fliessgeschwindigkeit von 1,3 ml/min über eine Lysin Sepharose® 4B (Pharmacia-LKB) Säule (ID = 2,6 cm; 58 ml Gel), die in einem Puffer bestehend aus 50 mM Natriumphosphat + 150 mM NaCl, pH 7,5 äquilibriert ist, gepumpt. Das ungebundene Material wird für die weitere IgA Isolierung verwendet.

(Das an die Säule gebundene Plasminogen kann nach einem weiteren Waschschritt mit 100 ml eines Puffers bestehend aus 50 mM Natriumphosphat, 0,5 M NaCl, 0,2 M Epsilon-Aminocapronsäure, pH 7,5 eluiert werden.)

Das Lysin Sepharose-ungebundene Material wird durch eine Fällung mit Dextransulfat (Sigma; Natrium Salz; MW: ca. 5000) von Lipoproteinen befreit: Dazu werden pro ml Probe 0,08 ml 10% Dextransulfatlösung und 1 ml 1 M Calciumchloridlösung zugesetzt. Die Mischung wird 30 min bei 25 - 30°C gerührt und anschliessend abzentrifugiert (18900 x g; 15 min; 4°C). Der Niederschlag wird verworfen und der Überstand wird auf einem Amicon (Beverly, MA, USA) Spiralmodul S1Y30-Cross Flow zur Entfernung der Calciumionen gegen das dreifache Probenvolumen an 0,9 % NaCl Lösung bei Raumtemperatur dialysiert.

Das dialysierte Material wird mit festem Ammoniumsulfat bei 4°C unter Rühren bis zu einer Konzentration von 2 M versetzt. Dann wird noch 30 min gerührt und anschliessend wird das gebildete Präzipitat 20 min mit 1540 x g bei 4°C abzentrifugiert. (Der Überstand wird verworfen.)

Der Niederschlag wird in Phosphat-gepufferter isotoner Kochsalzlösung pH 7,4 (in der Folge kurz PBS genannt) resuspendiert und auf einem Amicon Spiralmodul S1Y30 Cross Flow bei Raumtemperatur gegen das dreifache Probenvolumen an PBS dialysiert. Das dialysierte und durch Zentrifugation geklärte Material wird auf einer Hydroxyapatitsäule (BioRad, Richmond, CA, USA; Macro Prep Ceramic Hydroxyapatit; 20 Microns; 50 ml Gel; ID = 2,6 cm) aufgetrennt. Dabei werden pro Lauf 200 ml Probe bei einer Fliessgeschwindigkeit von 2 ml/min auf die mit Puffer A (PBS, pH 7,4) äquilibrierte Säule aufgetragen. IgA bindet an den Hydroxyapatit und kann mit 140 ml Puffer B (15 mM NaHPO₄/Na₂HPO₄ in PBS mit einem pH von 6,8) eluiert werden.

Das mit Puffer B vom Hydroxyapatit eluierte Material wird auf einem Amicon Spiralmodul S1Y30 Cross Flow bei Raumtemperatur gegen das dreifache Probenvolumen 50 mM Natriumacetat/Essigsäure Puffer, pH 5,0, dialysiert.

Das dialysierte Material wird mit einem Anionenaustauscher im Batch-Verfahren versetzt. Dazu wird der Ionenaustauscher (Fractogel® EMD TMAE 650(S); Korngrösse 0,02-0,04 mm; Merck, Darmstadt, Deutschland; 1:2 suspendiert in 50 mM Natriumacetat/Essigsäure Puffer, pH 5,0) so zur Probe zugesetzt, dass pro 10 mg Protein 2 ml Gel-Suspension vorliegen. Die Suspension wird über Nacht bei 4°C gerührt. Ungebundenes Material wird über eine Nutsche abgetrennt und das Gel 2 x mit 50 mM Natriumacetat/Essigsäure Puffer, pH 5,0 gewaschen. Anschliessend wird das Gel 2 Std. bei 4°C mit 50 mM Natriumacetat/Essigsäure Puffer + 80 mM NaCl, pH 6.0 gerührt. Dann wird das Gel über eine Nutsche von den durch diesen Puffer eluierten Proteinen abgetrennt.

Das eluierte Material wird in einer Amicon Rührzelle über eine Diaflo Ultrafiltrationsmembran YM30 auf 10 mg Protein/ml ankonzentriert.

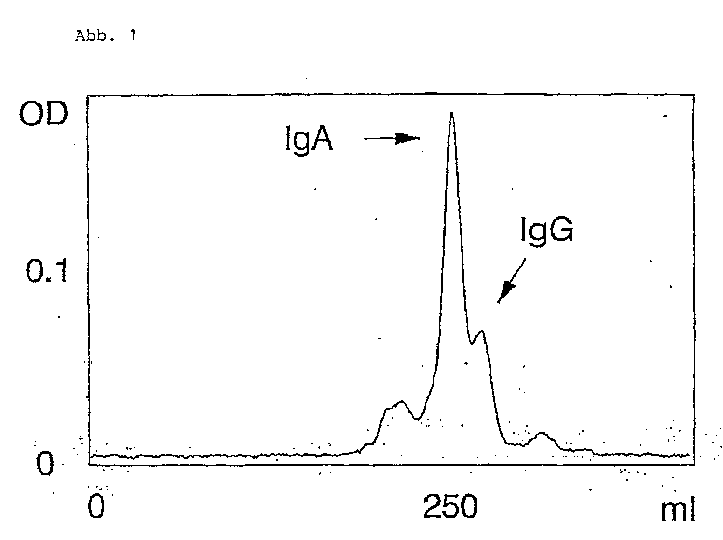

Dieses Material wird über eine Gelfiltrationssäule Superdex® 200 Prep Grade HR 35/600 (Pharmacia-LKB) erfindungsgemäss aufgetrennt: Pro Lauf werden 10 ml der Probe auf das in PBS, pH 7,4 äquilibrierte Gel bei einer Fliessgeschwindigkeit von 2,5 ml/min aufgetragen und isokratisch eluiert. Mit Hilfe dieser Säule kann ein Grossteil des noch vorhandenen IgG und anderer Verunreinigungen vom IgA abgetrennt werden (Abb. 1 zeigt die Gelfiltration auf Superdex® 200 HR 35/600). Die IgA-haltigen Fraktionen werden gesammelt, vereinigt und weiter mit Protein G Sepharose 4 Fast Flow (Pharmacia-LKB; in PBS, pH 7,4, äquilibriert) behandelt. Diese Affinitätschromatographie wird im Batch-Verfahren durchgeführt, wobei pro 50 ml Probe 1 ml Gel eingesetzt wird. Die Suspension wird über Nacht bei 4°C gerührt. Das Gel wird durch Zentrifugation von der Probe abgetrennt und 2x mit PBS, pH 7,4 gewaschen. Überstand und Waschlösung werden vereint und in einem Dialyseschlauch (Spectrapor MWCO 12.000 - 14.000) gegen destilliertes Wasser dialysiert.

### Das so hergestellte Endprodukt kann nun analysiert werden:

Die Analyse über Gelfiltration wird auf einer analytischen Superdex® 200 HR 10/30 FPLC-Säule (Pharmacia-LKB) mit PBS als Laufpuffer und einer Fliessgeschwindigkeit von 0,5 ml/min auf einer FPLC Anlage (Pharmacia-LKB) durchgeführt. Die optische Dichte (OD) wird bei 280 nm in einer Durchflussküvette gemessen und gegen das Elutionsvolumen (ml) aufgezeichnet.

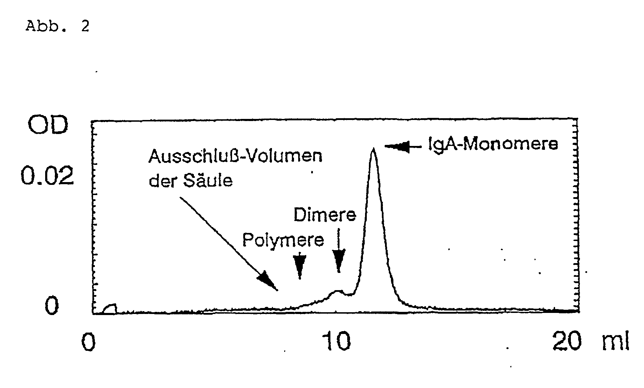

Das Ergebnis (Abb. 2) zeigt, dass das Endprodukt grösstenteils aus IgA-Monomeren und einem Anteil von 5-10 % IgA-Dimeren besteht, die sich in Lösung aus IgA-Monomeren bilden.
IgA-Aggregate liegen zu <2% vor. In einer auf >5 mg/ml ankonzentrierten IgA Lösung kann IgG durch Radialimmundiffusion nicht nachgewiesen werden, das bedeutet auf Grund des Sensitivitätslimits, dass weniger als 0,025 mg/ml IgG vorhanden sind. Das in erfindungsgemässer Weise hergestellte Endprodukt enthält also weniger als 1% IgG. Plasminogen und Fibrinogen sind ebenfalls nicht nachweisbar (Tabelle 1). Die Ausbeute an IgA in Bezug auf das Startmaterial (die NaCl-extrahierte Paste) beträgt 7 bis 12%.

### Tabelle 1

Das Ergebnis der Reinigung von IgA (Ausgangsmaterial ist die durch 12% Äthanol aus Cohnfraktion II+III gefällte und mit 0,9% NaCl extrahierte Paste)

Die Analyse von IgA, IgG und Plasminogen erfolgt durch Radialimmundiffusion (Mancini G., Carbonara A.O., Heremans J.F., Immunochemistry 2 [1965] 235-254); Fibrinogen wird durch die Methode von Ouchterlony (Acta Path. Microbiol. Scand. 26 [1949] 507-515) nachgewiesen.

| | IgA mg/ml | IgG mg/ml | Plasminogen mg/ml | Fibrinogen |
|---|---|---|---|---|
| Startmaterial | 1,3 | 1,70 | 0,41 | +++ |
| Endprodukt | 5,4 | n.n. | n.n. | n.n. |

| | | | | |
|---|---|---|---|---|
| n.n. = nicht nachweisbar.. | | | | |

### BEISPIEL 2

Wie in Beispiel 1 beschrieben, wird aus der durch 12 % Äthanol aus Cohnfraktion II+III gefällten Paste durch Rühren in 0,9 % NaCl ein Extrakt hergestellt.

Der noch trübe Extrakt wird mit festem Ammonsulfat gefällt. Die Endkonzentration der Lösung ist 2 M. Das Präzipitat wird 10 min mit 1540 x g bei 4°C abzentrifugiert. Der Überstand wird verworfen. Der Niederschlag wird in PBS gelöst und auf das Ausgangsvolumen aufgefüllt.

Anschliessend wird eine Dialyse (Dialyseschlauch: Fa. Spectrapor, MWCO 12-14 kD) gegen 0,9 % NaCl Lösung durchgeführt. Das dialysierte Material wird durch Zentrifugation und Filtration durch ein 0.2 mm Filter geklärt.

Die so vorbehandelte Probe (2 ml Aliquote) wird auf einer Superdex® 200 Prep Grade HR 16/60 Säule erfindungsgemäss aufgetrennt und die IgA enthaltenden Fraktionen isoliert.

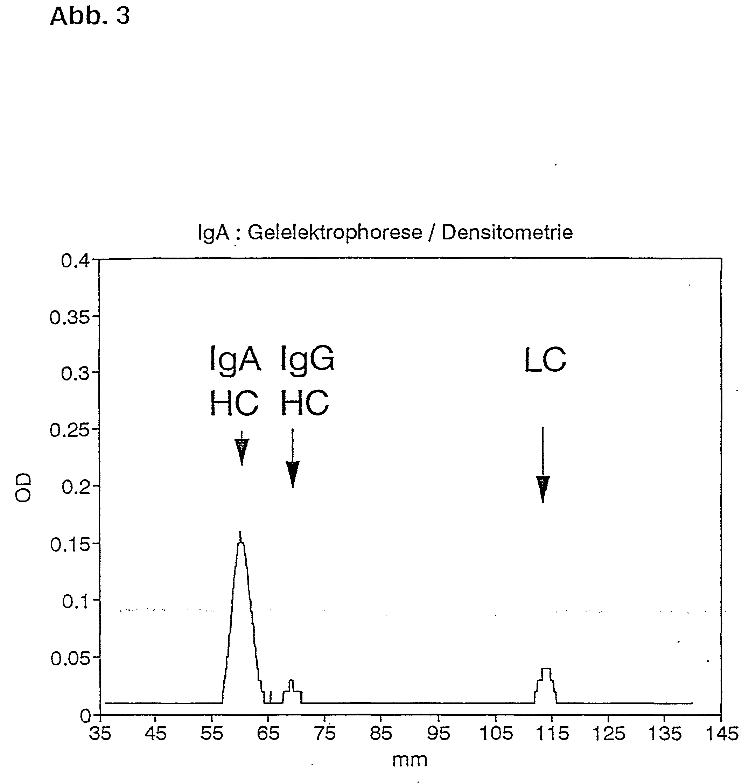

Die erfindungsgemäss isolierte Probe wird im reduzierten Zustand auf einer SDS-Gelelektrophorese nach dem System von Laemmli (Nature 227 [1970] 680-685) analysiert. Das Gel wird einer Proteinfärbung mit Comassie Brilliant Blue unterworfen und mit einem Video-Densitometer (BIORAD) ausgewertet. Die Intensität (OD) der Banden wird gegen die Wanderungsweite im Gel (mm) aufgezeichnet. Die Hauptbanden stellen die schweren und die leichten Ketten von IgA dar (Abb. 3 zeigt die densitometrische Auswertung der SDS-Gelektrophorese; "LC" = light chain [leichte Kette], "HC" = heavy chain [schwere Kette]). Geringe Mengen der schweren Kette von IgG (<10 % von IgA) werden beobachtet. Diese Reste von IgG können durch eine anschliessende Protein-G-Sepharose-Behandlung entfernt werden.

Diese Methode erlaubt die Isolierung von IgA nach Klärung des Ausgangsmaterials direkt über die erfindungsgemässe Gelfiltrationsmethode.

Die Ausbeute am IgA in Bezug auf das Ausgangsmaterial (NaCl extrahierte Paste) beträgt etwa 38 %.

### BEISPIEL 3

Als Ausgangsmaterial für die IgA-Isolierung wird ein Pool (mindestens 1000 Spender) von humanem Plasma verwendet.

800 ml dieses bei -20°C gelagerten Plasma-Pools werden aufgetaut, der Niederschlag (Kryopräzipitat) abgetrennt und der Überstand durch hochtourige Zentrifugation geklärt. Der geklärte Überstand wird mit dem gleichen Volumen an 14%igem Polyethylenglycol (PEG 6000) (14 % (w/v) in 0,100M Tris, 0,150M NaCl, pH 8,0) versetzt und 16 h bei 4°C gerührt. (Die Endkonzentration beträgt 7 % PEG.) Dann wird 20 min abzentrifugiert (18900 x g, 4°C). Der Überstand wird mit 21% (w/v) PEG 6000 in 0,10M Tris-HCl, 0,150M NaCl-Puffer, pH 6,5) auf eine Endkonzentration von 14 % PEG gebracht und wieder 16 h bei 4°C gerührt. Dann wird abermals abzentrifugiert (6000 x g, 20 min, 4°C). Der Niederschlag wird mit 300 - 350ml 0,1M Tris, 0,15M NaCl-HCl-Puffer (pH 8,0) gelöst und 4x gegen je 5 l bidestilliertes Wasser dialysiert (4°C, Dialyseschlauch mit 50 Kilo-Dalton (kD) Trenngrenze). Das Dialysat wird durch Zentrifugation geklärt. Niedergeschlagenes Material wird verworfen.

Der Überstand dieser Zentrifugation wird mit 3 M Ammoniumsulfat, 0,040 M Tris, Essigsäure, pH=7,6, auf eine Endkonzentration von 2 M Ammoniumsulfat gebracht. Dann wird 1h bei 4°C gerührt und abermals abzentrifugiert (19800 x g, 20 min, 4°C). Der Niederschlag wird in 150 - 170 ml NaCl gelöst und 2x 16h bei 4°C in einem Dialyseschlauch mit 12- 14 kD dialysiert.

Trenngrenze gegen je 5 l isotone Kochsalzlösung dialysiert. Durch langsame Zugabe von 2,5 M ZnSO₄ (in destilliertem Wasser gelöst) zum Dialysat wird die Zinksulfatkonzentration in dieser Lösung auf 0,1 M gebracht. Gleichzeitig wird durch Zugabe von 1 M Na₂CO₃ in destilliertem Wasser der pH auf 6 - 8 (vorzugsweise 7) gehalten. Dann wird 2 h bei 25°C gerührt und abzentrifugiert. Der Niederschlag wird verworfen. Der Überstand wird 3x bei 4°C gegen bidestilliertes Wasser dialysiert.

Dieses Material (in Tabelle 2 als "Zinksulfat-Überstand" bezeichnet) enthält etwa gleiche Mengen IgA und Haptoglobin und kleine Reste IgG.

**Tabelle 2**

| | | | | | |
|---|---|---|---|---|---|
| Die prozentuelle Verteilung von IgG, IgA, IgM und Haptoglobin (Hp) in den Produkten der einzelnen Reinigungsschritte (bezogen auf Gesamtprotein), bestimmt durch Radialimmundiffusions-Analyse, und die Ausbeute (in % des Ausgangsmaterials) von IgA. | | | | | |
| Die Bereiche, ermittelt in bis zu 5 getrennt durchgeführten Versuchen, sind angeführt. | | | | | |

| | Ausbeute | | | | |
|---|---|---|---|---|---|
| | % IgG | IgA % | % IgM | % Hp | % IgA |
| Ausgangsmaterial Plasma (Kryo-Überstand) | 11-18 | 2-4 | 0,7-2,0 | 2-4 | 100 |
| 14 % PEG-Niederschlag | 20-30 | 10-15 | bis 0,5 | 5-10 | 57-75 |
| 2 M Ammonsulfat-Niederschlag | 20-32 | 12-20 | bis 0,5 | 10-14 | 40-60 |
| 0,01 M Zinksulfat-Überstand | 2-5 | 30-50 | bis 0,5 | 30-50 | 10-20 |
| Rivanol-Überstand | <15 | >85 | n.n. | n.n. | 4-5 |
| nach Superdex® 200 | <2 | >98 | n.n. | n.n. | 2-3 |

| | | | | | |
|---|---|---|---|---|---|
| n.n. = nicht nachweisbar. | | | | | |

Das dialysierte Material nach der Zinksulfatfällung wird mit 1 % Rivanol (6,9-Diamino-2-ethoxyacridinlaktat in bidestilliertem Wasser gelöst) auf eine Endkonzentration von 0,1 % (w/v) Rivanol gebracht. Im Lauf der Reaktion sinkt der pH (durch Milchsäure-Freisetzung) ab. Mit 0,01 M NaOH hält man den pH der Mischung auf 7,8 - 8,0. Anschliessend wird 1 h bei Raumtemperatur gerührt und dann abzentrifugiert. Der Niederschlag wird verworfen. Der Überstand wird 3 bis 6x bei 4°C gegen bidestilliertes Wasser dialysiert. Das Dialysat wird über Ultrafiltration (vorzugsweise in einer Amicon-Rührzelle mit Amicon-YM10-Membran, Amicon, Beverly, MA, USA) ankonzentriert.

Der dialysierte Rivanol-Überstand wird einer Batch-Behandlung mit einem Kationentauscher (S-Sepharose® Fast Flow, Pharmacia-LKB) unterworfen:

Vorbehandlung der S-Sepharose® Fast Flow: 4 ml der Gelsuspension werden entnommen, mit 6 ml 0,2 M Tris/HCl-Puffer pH 8,0 versetzt und abzentrifugiert. Der Gelkuchen wird durch Resuspension in 50 ml 0,01 M Tris/HCl-Puffer pH 8,0 und Zentrifugation gewaschen und als Suspension (1 Teil Gel, 1 Teil Puffer) in 0,01 M Tris/HCl-Puffer pH 8,0 aufgehoben. Vor der Verwendung wird ein Teil der Suspension abzentrifugiert, der Überstand verworfen und der so erhaltene Gelkuchen für Adsorptionsversuche eingesezt:

5 ml Rivanol-Überstand werden mit 2 M Tris-Base auf pH 8 titriert. Dieses Material wird auf 0,5 ml S-Sepharose® Fast Flow Gel pipettiert. Nach 2 h Inkubation bei 4°C (unter Schütteln) wird abzentrifugiert und der Überstand über Gelfiltration weiter gereinigt: 2-ml-Aliquote des Überstands werden auf einer Superdex® 200 Prep Grade HR 16/60-FPLC-Säule (Pharmacia-LKB) chromatographiert. Die IgA-hältigen Fraktionen werden gesammelt und gegen Wasser dialysiert. Dieses Material enthält laut Radialimmundiffusions-Analyse zu mindestens 98 % IgA. IgG und Haptoglobin konnten nicht nachgewiesen werden (<2%).

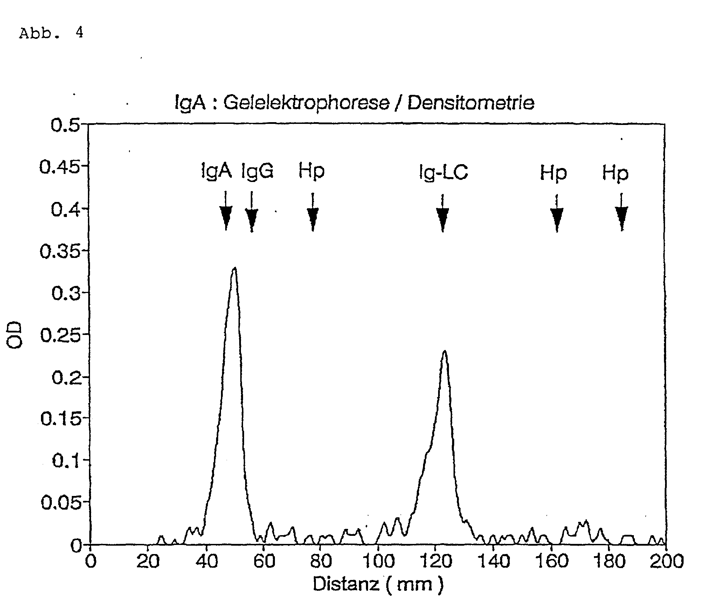

Auch eine SDS-Polyacrylamid-Elektrophorese (reduziert, nach dem System von Laemmli) mit nachfolgender Densitometrie (in Beispiel 2 beschrieben) zeigt nur noch die Banden für die schweren und die leichten Ketten von IgA (Abb. 4, "IgA" bzw. "IgG" bezeichnen die Positionen der schweren Ketten von IgA bzw. IgG; "Ig-LC" bzw. "Hp" bezeichnen die Positionen der leichten Ketten der Immunglobuline bzw. der Haptoglobin-Untereinheiten).

### BEISPIEL 4

Wie in der Patentanmeldung EP 0506 651 beschrieben, wird aus humanem Plasma eine Cohn II+III-Fraktion hergestellt, die mit einem Phosphat-Acetat-Puffer extrahiert wird. Dieses Material wird mit Ethanol bei einem pH-Wert von 5,3 und bei -2°C auf eine Konzentration von 12 % versetzt, wobei sich ein Niederschlag bildet, der abgetrennt wird. Der Überstand wird, wie beschrieben, mit einem Anionentauscher behandelt.

Das Anionentauschermaterial mit den daran gebundenen Proteinen wird nun erfindungsgemäss weiter behandelt: 100 g Paste werden in 1000 ml dest. Wasser suspendiert und durch eine Sinter-Nutsche gezogen.

Das zurückgehaltene Gelmaterial wird mit 2 l dest. Wasser gewaschen.

Das gewaschene Gelmaterial wird mit 300 ml 0,5 M NaCl (in dest. Wasser gelöst) versetzt, 10 min bei 4°C inkubiert und dann abzentrifugiert.

Der Überstand (in Tabelle 3 als "NaCl-Extrakt" bezeichnet) wird abgehoben und steril filtriert.

Der Extrakt wird mit einem gleich grossen Volumen einer Lösung von 2 M Ammoniumsulfat, 0,1 M Natriumacetat, pH 6,0, versetzt und diese Mischung, die nun 1 M an Ammoniumsulfat ist, auf eine thiophile Chromatographie-Säule aufgetragen.

Die Säule (Typ XR-16/20 von Pharmacia-LKB) ist mit 30 ml Affi-T Thiophilic Agarose (Kem-En-Tee, Kopenhagen, Dänemark) gefüllt und mit einer Lösung von 1 M Ammoniumsulfat, 0,05 M Natriumacetat pH 6,0 equilibriert.

Mit einer Geschwindigkeit von 0,8 - 1,0 ml/min wird die Mischung über die Säule gepumpt. Nicht gebundene Proteine werden mit 120 ml 1 M Ammoniumsulfat, 0,04 M Acetat pH 6,0 ausgewaschen, dann wird mit 120 ml 0,6 M Ammoniumsulfat, 0,03 M Acetat pH 6,0 eine IgA-hältige Fraktion eluiert. Restliche noch gebundene Proteine werden mit 120 ml 50 mM TRIS, pH 8 ausgewaschen, bevor die Säule wieder in 1 M Ammoniumsulfat, 0,05 M Acetat reequilibriert wird.

Die mit 0,6 M Ammoniumsulfat eluierte Fraktion wird 3x gegen dest. Wasser in einem Dialyseschlauch mit einem Cut-off von 50 kD dialysiert.

Das dialysierte Material (in Tabelle 3 als "Ammoniumsulfat-Eluat" bezeichnet) wird über Ultrafiltration auf etwa 3 mg/ml ankonzentriert und dann in 10-ml-Aliquoten über eine Gelfiltrationssäule (Superdex® S200 Prep Grade HR 35/600, Pharmacia-LKB) chromatographiert.

Die IgA-hältigen Fraktionen werden gesammelt, gepoolt und mehrmals gegen PBS dialysiert.

Die Ausbeute an IgA, gemessen am IgA-Gehalt des "NaCl-Extrakts", beträgt etwa 22 - 30 % (Tabelle 3).

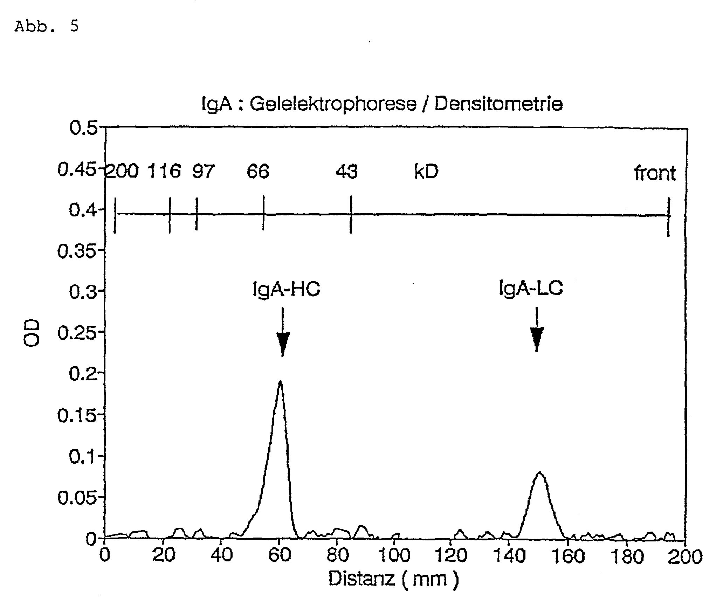

Das erfindungsgemäss gereinigtes Material besteht bis zu 95 % aus IgA, wie durch Radialimmudiffusions-Analyse gemessen wurde (Tabelle 3). Abbildung 5 zeigt die SDS-Polyacrylamid-Gelelektrophorese der reduzierten Probe nach dem Laemmli-System und nachfolgende Densitometrie (Beschreibung in Beispiel 2) des Gels. Nur die charakteristischen Banden für die schweren ("IgA-HC") und die leichten ("IGA-LC") Ketten des IgA sind erkennbar.

IgA mit einer Reinheit von >99 % kann durch eine weitere Behandlung mit Protein-G-Sepharose Fast Flow (Pharmacia-LKB) im Batchverfahren isoliert werden.

Die Protein-G-Sepharose-Menge ist dabei so zu bemessen, dass 1 ml Gelmaterial pro 10 mg noch enthaltenes IgG im Batch vorhanden ist. Nach 3 -6stündiger Inkubation unter Schütteln wird die Protein-G-Sepharose mit dem daran gebundenen IgG durch Zentrifugation abgetrennt. Der Überstand wird gegen destilliertes Wasser dialysiert.

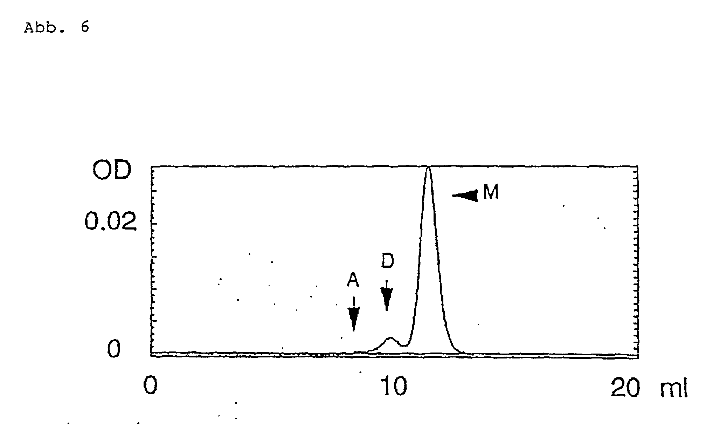

Dieses Endprodukt wurde auch auf einer analytischen Superdex® 200 HR 10/30 Gelfiltrationssäule (Methodenbeschreibung in Beispiel 1) analysiert. Es besteht zum grössten Teil aus IgA-Monomeren mit kleinen Mengen (5 - 10 %) IgA-Dimeren (Abb. 6; "D" = IgA-Dimere, "M" = IgA-Monomere, "A" = IgA-Aggregate). IgA-Aggregate liegen, wenn überhaupt, in einer Menge von <2 % vor. In dem erfindungsgemäss hergestellten Endprodukt kann durch Radioalimmundiffusion kein IgG mehr nachgewiesen werden. Auf Grund des Sensibilitätslimits der angewendeten Methode bedeutet das, dass weniger als 1 % IgG in der IgA-Präparation vorhanden ist.

**Tabelle 3**

| | | | |
|---|---|---|---|
| Die prozentuelle Verteilung von IgG und IgA in den Produkten der einzelnen Reinigungsschritte (bezogen auf Gesamtprotein), bestimmt durch Radial-Immundiffusions-Analyse, und die Ausbeute (in % des Ausgangsmaterials) von IgA. Die Bereiche, ermittelt in 4 getrennt durchgeführten Versuchen, sind angeführt. | | | |

| | % IgG | % IgA | % IgA Ausbeute |
|---|---|---|---|
| NaCl-Extrakt | 6 - 9 | 17 - 26 | 100 |
| Ammoniumsulfat-Eluat | 5 - 12 | 70 - 85 | 35 - 47 |
| nach Superdex® 200 | 4 - 9 | 91 - 95 | 22 - 30 |
| Endprodukt | n.n. | >99 | 18 - 25 |

| | | | |
|---|---|---|---|
| n.n. = nicht nachweisbar | | | |

### BEISPIEL 5

Die IgA-Subklassen-Zusammensetzung und die Verteilung von Kappa- und Lambda-Leichtketten in den gereinigten IgA-Präparaten

Die erfindungsgemäss in Beispiel 1, 3 und 4 hergestellten IgA-Präparationen wurden mit Hilfe von IgA-Subklassen-spezifischen Radialimmundiffusionsplatten (The Binding Site, Birmingham, England) auf ihre IgA-Subklassen-Zusammensetzung getestet (Methode nach der Vorschrift der Fa. Binding Site) und mit einem Plasma-Pool (aus mehr als 1000 gesunden, erwachsenen Blutspendern gebildet) verglichen: Die Zusammensetzung der IgA-Subklassen in den erfindungsgemäss hergestellten Produkten unterschied sich kaum von der des Normalplasmas (Tabelle 4).

Die Leichtkettenzusammensetzung des gereinigten IgA wurde mit Radialimmundiffusionsplatten der Firma Behring (Marburg, Deutschland) untersucht (Methode nach der Vorschrift der Fa. Behring). Es konnten keine Unterschiede im Kappa:Lambda-Verhältnis zwischen gereinigtem IgA und einem Standard-Plasma-Pool (in dem das Kappa:Lambda-Verhältnis aller Immunglobulinklassen gemessen wurde) beobachtet werden (Tabelle 5).

Diese Ergebnisse zeigen, dass das erfindungsgemäss hergestellte IgA in seiner Zusammensetzung dem im Serum enthaltenen IgA entspricht.

**Tabelle 4**

| Die IgA-Subklassenzusammensetzung der gereinigten IgA-Präparate, gemessen durch radiale Immundiffusion | | |
|---|---|---|
| | % IgA1 | % IgA2 |
| Plasma-Pool | 79 | 21 |
| IgA aus Beispiel 1 | 78 | 22 |
| IgA aus Beispiel 3 | 76 | 24 |
| IgA aus Beispiel 4 | 75 | 25 |

**Tabelle 5**

| Die Leichtkettenzusammensetzung von gereinigtem IgA, gemessen durch radiale Immundiffusion (Auswertung in Units/dl) | |
|---|---|
| | Kappa/Lambda |
| Plasma-Pool | 0,99 |
| IgA aus Beispiel 3 | 0,95 |

### BEISPIEL 6

### Die Austestung der biologischen Wirksamkeit der gereinigten IgA-Präparate

Die Fähigkeit der IgA-Produkte, nach ihrer erfindungsgemässen Reinigung Bakterien zu erkennen und zu binden, wird in einem Testsystem untersucht:

Von einer E. coli-Suspension (Nr. COPO54, 1,39 x 1010/ml) werden 20-ml-Aliquote entnommen und in 1,4 ml erfindungsgemäss hergestelltem IgA (1 mg/ml) bzw. als Kontrolle in 1,4 ml human Plasma-Pool (1 mg/ml IgA) suspendiert. 0,1 ml einer 1%(w/v)-Lösung von bovinem Serumalbumin in PBS (in der Folge kurz PBS/BSA genant) werden zugegeben. Es wird 60 min bei 4°C unter gelegentlichem Schütteln inkubiert. Dann wird abzentrifugiert (Eppendorf, 12000 rpm, 5 min, 4°C) und die Bakterien werden 4x mit je 1 ml PBS/BSA gewaschen. Die Bakterien werden in 0,5 ml PBS/BSA resuspendiert. Diese Suspensionen werden mit je 20 ml eines fluoreszenzmarkierten Anti-human-IgA-Antikörpers (Fluorescein (DTAF) conjugated Affi pure rabbit F(ab)2 anti human IgA (alpha chain spec.), Jackson ImmunoResearch, West Grove, PA, USA) versetzt und 60 min bei 4°C unter gelegentlichem Schütteln inkubiert. Dann wird 4x mit je 1 ml PBS/BSA gewaschen, in je 0,5 ml PBS/BSA suspendiert. Die Bindung von IgA und fluoreszenz-markiertem Anti-human-IgA an die Bakterien wird in einem Fluorescence-activated-Cell-Sorter (Typ: FACStar-Plus, Becton Dickinson, Mountain View, CA, USA) gemessen.

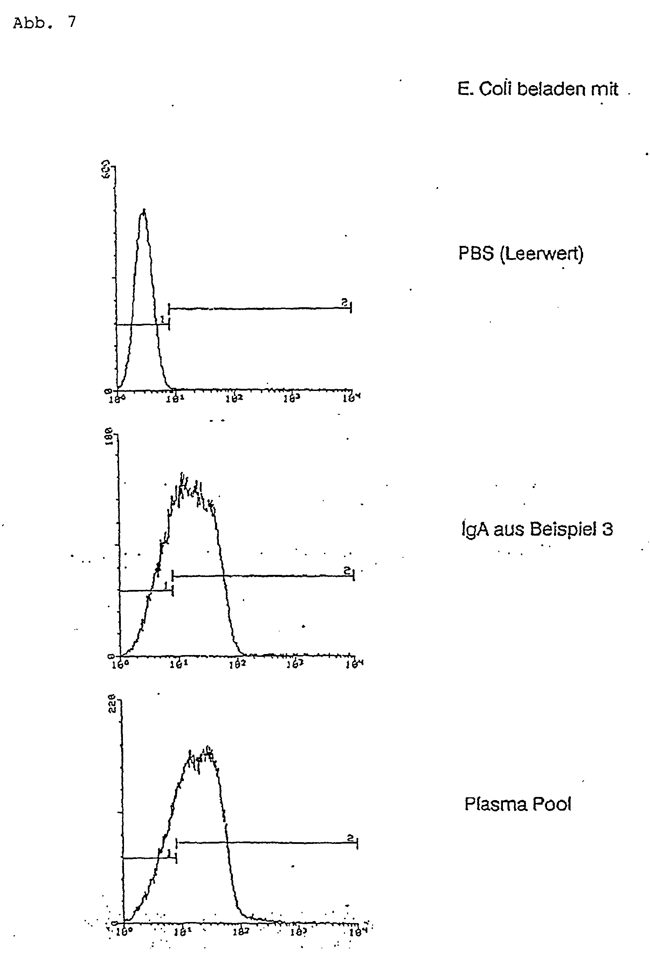

Als Leerwert dienen Bakterien, die zwar mit dem fluoreszenzmarkierten Antikörper, nicht aber mit IgA-hältigem Produkt (sondern statt dessen mit reinem PBS) inkubiert wurden. Die Analyse zeigt (Abb. 7, die x-Achse definiert die Intensität der Fluoreszenz und die y-Achse die Anzahl der detektierten Bakterien), dass das erfindungsgemäss isolierte IgA an Bakterien gleich gut bindet wie das IgA aus dem Plasma-Pool. 74 % der Bakterien, die mit Plasma-IgA und 77 % der Bakterien, die mit erfindugsgemäss gereinigtem IgA inkubiert waren, haben IgA gebunden.

Dieses Ergebnis zeigt, dass die biologische Funktion des IgA, an bestimmte Bakterien zu binden, auch nach der erfindungsgemässen Reinigung erhalten bleibt.

### BEISPIEL 7

### Lyophilisation und Hitzebehandlung des isolierten IgA zur Inaktivierung eventuell vorhandener viraler Verunreinigungen

4 ml eines erfindungsgemäss hergestellten IgA-Präparats mit einer IgA-Konzentration von 10 mg/ml werden lyophilisiert. Anschliessend wird das gefriergetrocknete Material im Lyophilisationsfläschchen befeuchtet (durch Zugabe von 7 % Wasser im Vergleich zum Gesamtgewicht der Probe). Danach erfolgt eine Hitzebehandlung von 10 h bei 60°C sowie anschliessend 1 h bei 80°C im dicht verschlossenen Gefäss.

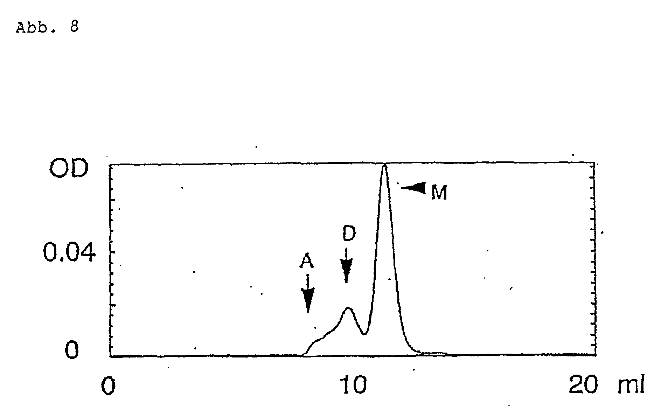

Die behandelten Proben werden mit PBS auf das Ausgangsvolumen rekonstituiert und die so erhaltenen Lösungen über Gelfiltration (beschrieben in Beispiel 1) auf einer Superdex^{R} S200 HR 10/30 FPLC-Säule analysiert. Abbildung 8 ("M" = IgA-Monomere, "D" = IgA-Dimere, "A" = IgA-Aggregate) zeigt, dass auch nach Lyophilisation und Hitzebehandlung das IgA zu mehr als 96 % aus IgA-Monomeren (inklusive IgA-Dimeren) besteht.

### BEISPIEL 8

### Hitzebehandlung von gelöstem IgA

Erfindungsgemäss isoliertes IgA wird in destilliertem Wasser in einer Konzentration von 0,4 mg/ml gelöst und in einem Wasserbad bei unterschiedlichen Temperaturen unterschiedlich lange inkubiert.

Anschliessend wird das Material auf einer Superdex® 200 HR 10/30 Gelfiltrationssäule, wie in Beispiel 1 beschrieben, analysiert.

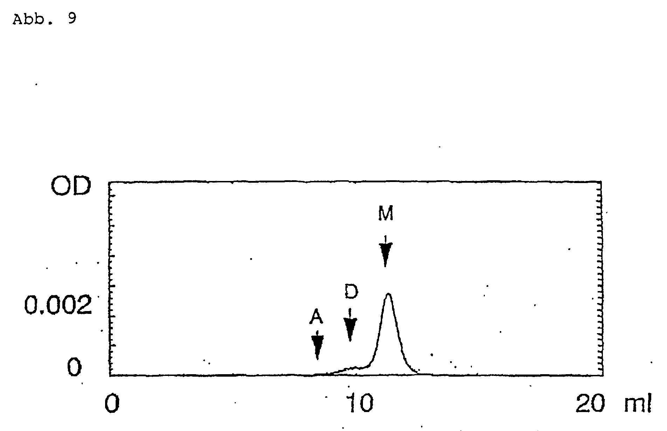

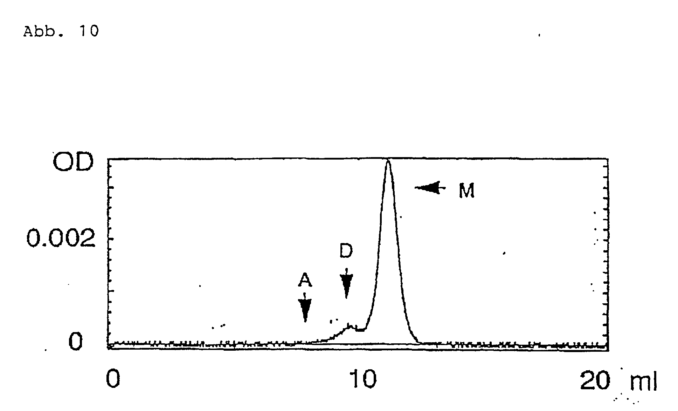

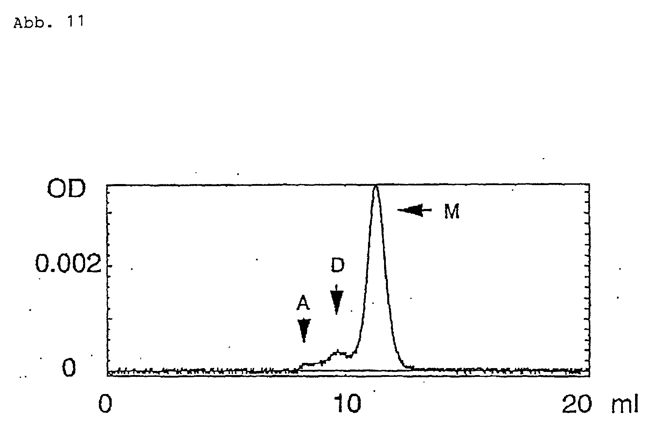

Der Vergleich mit einer unbehandelten IgA-Präparation (siehe Abb. 2 oder Abb. 6) zeigt, dass weder bei einer Erhitzung auf 60°C während 30 min (Abb. 9), noch bei einer Erhitzung auf 40°C während 8 Std. (Abb. 10) Aggregate gebildet werden. Selbst bei einer 8stündigen Erwärmung auf 40°C und einer nachfolgenden 8stündigen Erhitzung auf 50°C (Abb. 11) werden weniger als 2 % Aggregate beobachtet. In den Abbildungen 9-11 bezeichnen "M", "D" bzw. "A" die Positionen der IgA-Monomere, IgA-Dimere bzw. IgA-Aggregate.

### BEISPIEL 9

Eine im wesentlichen aus IgA und IgG bestehende Probe wird auf vom Material her unterschiedlichen, aber gleich dimensionerten Gelfiltrationssäulen analysiert, um den Einfluss von hydrophoben und/oder elektrostatischen Wechselwirkungen mit den Säulenmaterialien zu testen. Dazu werden gleich grosse Aliquote auf die Säulen aufgetragen und mit identischen Elutionsbedingungen (0,5 ml/min) isokratisch eluiert.

Die folgenden Analysen wurden in Gelfiltrationssäulen, die mit Phosphat gepufferter 0,15-M-NaCl-Lösung äquilibriert waren, durchgeführt (Fliessgeschwindigkeit: 0,5 ml/min, Messung der optischen Dichte (OD) bei 280 nm):

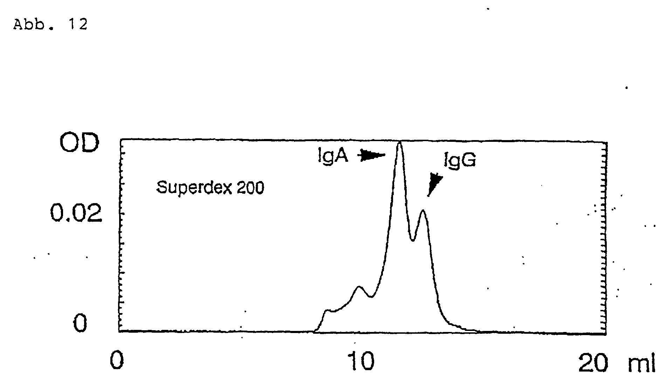

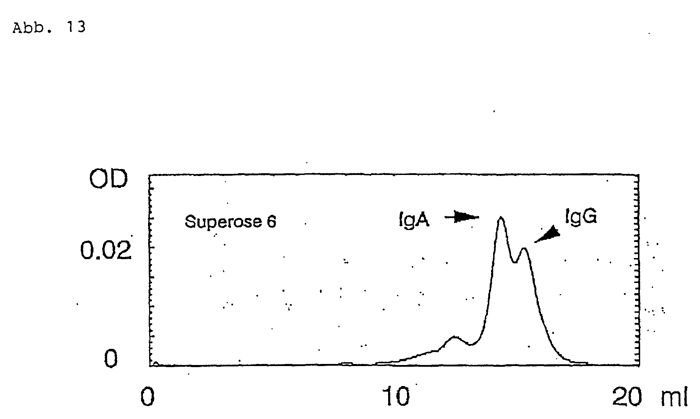

Auf einer Superdex® 200 HR 10/30 Säule (Pharmacia-LKB) werden IgG und IgA ebenso getrennt (Abb. 12) wie auf einer Superose® 6 HR 10/30 Säule (Pharmacia-LKB) (Abb. 13).

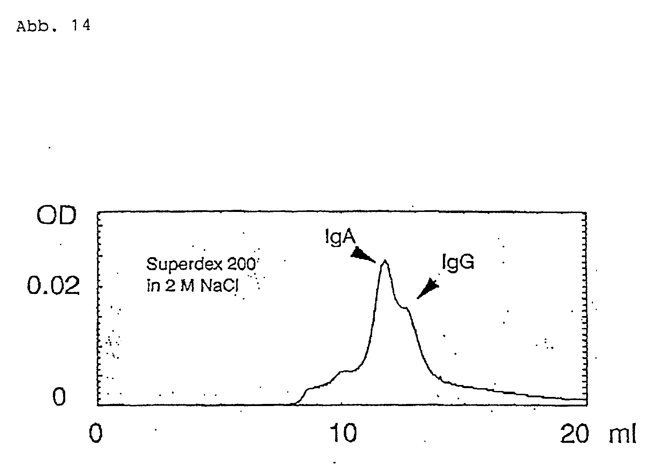

Wird die Gelfiltration unter Bedingungen hoher Ionenstärke (2 M NaCl) durchgeführt, so ist auch auf der Superdex® 200 HR 10/30 Säule keine klare Trennung mehr möglich, da die Protein-Peaks zusammenwandern (Abb. 14). Dieses Ergebnis deutet darauf hin, dass die Trennung von IgA und IgG auf Superdex® 200 nicht nur auf dem Unterschied im Molekulargewicht (IgA: 162 kD, IgG: 153 kD [Heremans J.F., Immunoglobulin A, in: The Antigens, Vol. 2 (1974) S. 365-522; Academic Press, New York 1974]) beruhen, sondern auf unterschiedlichen hydrophoben und/oder elektrostatischen Wechselwirkungen von IgG und IgA mit dem Säulenmaterial.

## Patentansprüche

1. Stabiles pharmazeutisches Präparat bestehend aus wenigstens 70%, bevorzugt 90% humanem, monomeren IgA erhältlich durch
(a) Reinigung einer Immunglobulin-haltigen Fraktion mittels einer Gelpermeationschromatographie und/oder einer thiophilen Chromatographie, so dass ein monomeres IgA mit einem Anteil an IgG von weniger als 10 Gew.% am Gesamtimmunoglobulin erhalten wird.
(b) Durchführung eines Verfahrens zur Inaktivierung von Viren.

2. Stabiles pharmazeutisches Präparat nach Anspruch 1,
**dadurch gekennzeichnet, dass** als Verfahren zur Inaktivierung von Viren eine Hitzebehandlung durchgeführt wird.

3. Stabiles pharmazeutisches Präparat nach einem der Ansprüche 1 oder 2, **dadurch gekennzeichnet, dass** das IgA das IgA1 und IgA2 in einer nativen Zusammensetzung enthält.

4. Stabiles pharmazeutisches Präparat nach einem der Ansprüche 1 bis 3, **dadurch gekennzeichnet, dass** es frei von Fibrinogen ist.

5. Stabiles pharmazeutisches Präparat nach einem der Ansprüche 1 bis 4, **dadurch gekennzeichnet, dass** es frei von Plasminogen ist.

6. Stabiles pharmazeutisches Präparat gemäß einem der Ansprüche 1 bis 5 formuliert zur oralen oder mukosalen Verabreichung.

7. Stabiles pharmazeutisches Präparat nach einem der vorherigen Ansprüche, welches zusätzlich pharmazeutisch akzeptable Hilfs- und Zusatzstoffe enthält.

8. Verfahren zur Herstellung eines virussicheren stabilen pharmazeutischen Präparats bestehend aus wenigstens 70%, bevorzugt 90% humanem, monomeren IgA, welches die folgenden Schritte umfasst:
(a) Reinigung einer Immunglobulin-haltigen Fraktion durch eine Gelpermeationschromatographie und/oder eine thiophile Chromatographie durchgeführt, so dass ein monomeres IgA mit einem Anteil an IgG von weniger als 10 Gew.% am Gesamtimmunoglobulin erhalten wird.
(b) Durchführung eines Verfahrens zur Inaktivierung von Viren.

9. Verfahren nach Anspruch 8, **dadurch gekennzeichnet, dass** das Verfahren zur Inaktivierung von Viren ein Erhitzen des monomeren IgA umfasst.

10. Verfahren nach einem der Ansprüche 8 oder 9, **dadurch gekennzeichnet, dass** als Immunglobulin-haltige Fraktion humanes Blutserum, Plasma, Plasmafraktionen oder Colostrum verwendet wird.

11. Verfahren nach einem der Ansprüche 8 bis 10, **dadurch gekennzeichnet, dass** als Ausgangsmaterial die Cohn II + III Fraktion verwendet wird.

12. Verfahren nach Ansprüche 8 bis 11, **dadurch gekennzeichnet, dass** das Ausgangsmaterial vorgereinigt wird.

13. Verfahren nach Anspruch 8, **dadurch gekennzeichnet, dass** eine Gelpermeationschromatographie mit hydrophilen/hydrophoben bzw. elektrostatischen Wechselwirkungen vorgenommen wird.

14. Verfahren nach einem der Ansprüche 8 bis 13, **dadurch gekennzeichnet, dass** als weitere Reinigung eine Affinitätschromatographie durchgeführt wird.

15. Verfahren.nach einem der Ansprüche 8 bis 14, **dadurch gekennzeichnet, dass** als Verfahren zur Inaktivierung von Viren eine Hitzebehandlung zwischen 40°C und 80°C, am meisten bevorzugt zwischen 50°C und 65°C, über einen zur Inaktivierung von Viren ausreichend langen Zeitraum durchgeführt wird.

16. Verfahren nach einem der Ansprüche 8 bis 15, **dadurch gekennzeichnet, dass** eine Dialyse gegen Wasser vor einer Hitzebehandlung durchgeführt wird.

17. Verfahren nach Anspruch 8, **dadurch gekennzeichnet, daß** als Immunglobulin-haltige Fraktion Plasma oder eine Plasmafraktion verwendet wird und die Reinigung an einer thiophilen Matrix erfolgt.

## Claims

1. Stable pharmaceutical preparation consisting of at least 70 %, preferably 90 %, of human, monomer IgA which can be obtained by
(a) purifying an immunoglobulin-containing fraction by means of gel-permeation chromatography and/or thiophilic chromatography, so that a monomer IgA having a proportion of IgG of less than 10 wt.% of total immunoglobulin is obtained.
(b) Carrying out a process for inactivating viruses.

2. Stable pharmaceutical preparation according to claim 1,
**characterised in that** heat treatment is carried out as a process for inactivating viruses.

3. Stable pharmaceutical preparation according to one of claims 1 or 2, **characterised in that** the IgA contains the IgA1 and IgA2 in a natural composition.

4. Stable pharmaceutical preparation according to one of claims 1 to 3, **characterised in that** it is free of fibrinogen.

5. Stable pharmaceutical preparation according to one of claims 1 to 4, **characterised in that** it is free of plasminogen.

6. Stable pharmaceutical preparation according to one of claims 1 to 5 formulated for oral or mucosal administration.

7. Stable pharmaceutical preparation according to one of the previous claims, which additionally contains pharmaceutically acceptable auxiliaries and additives.

8. Process for producing a virus-safe, stable pharmaceutical preparation consisting of at least 70 %, preferably 90 %, of human, monomer IgA, which comprises the following steps:
(a) purifying an immunoglobulin-containing fraction by means of gel-permeation chromatography and/or thiophilic chromatography carried out, so that a monomer IgA having a proportion of IgG of less than 10 wt.% of total immunoglobulin is obtained.
(b) Carrying out a process for inactivating viruses.

9. Process according to claim 8, **characterised in that** the process for inactivating viruses comprises heating the monomer IgA.

10. Process according to one of claims 8 or 9, **characterised in that** human blood serum, plasma, plasma fractions or colostrum is used as the immunoglobulin-containing fraction.

11. Process according to one of claims 8 to 10, **characterised in that** the Cohn II + III fraction is used as the starting material.

12. Process according to claims 8 to 11, **characterised in that** the starting material is prepurified.

13. Process according to claim 8, **characterised in that** gel-permeation chromatography is carried out using hydrophilic/hydrophobic or electrostatic interactions.

14. Process according to one of claims 8 to 13, **characterised in that** affinity chromatography is carried out as further purification.

15. Process according to one of claims 8 to 14, **characterised in that** heat treatment between 40°C and 80°C, in most cases preferably between 50°C and 65°C, ' over a period of time sufficiently long to inactivate viruses is carried out as a process for inactivating viruses.

16. Process according to one of claims 8 to 15, **characterised in that** dialysis against water is carried out before heat treatment.

17. Process according to claim 8, **characterised in that** plasma or a plasma fraction is used as the immunoglobulin-containing fraction and purification takes place on a thiophilic matrix.

## Revendications

1. Préparation pharmaceutiquement stable, constituée d'au moins 70 %, de préférence de 90 % d'immunoglobuline A (IgA) humaine, obtenue par :
(a) épuration d'une fraction contenant de l'immunoglobuline, au moyen d'une chromatographie à perméation sur gel et/ou d'une chromatographie thiophile, de manière à obtenir une IgA monomère, ayant une proportion d'IgG inférieure à 10 % en poids sur l'immunoglobuline globale.
(b) exécution d'un procédé d'inactivation des virus.

2. Préparation pharmaceutiquement stable selon la revendication 1, **caractérisée en ce que** l'on effectue un traitement à la chaleur comme procédé d'inactivation des virus.

3. Préparation pharmaceutiquement stable selon l'une des revendications 1 ou 2, **caractérisée en ce que** l'IgA contient IgA1 et IgA2 en une composition native.

4. Préparation pharmaceutiquement stable selon l'une des revendications 1 à 3, **caractérisée en ce qu'**elle est exempte de fibrinogène.

5. Préparation pharmaceutiquement stable selon l'une des revendications 1 à 4, **caractérisée en ce qu'**elle est exempte de plasminogène.

6. Préparation pharmaceutiquement stable selon l'une des revendications 1 à 5, formulée pour l'administration orale ou mucosale.

7. Préparation pharmaceutiquement stable selon l'une des revendications précédentes, qui contient, en plus, des additifs et des adjuvants acceptables pharmaceutiquement.

8. Procédé de préparation d'une préparation pharmaceutique stable, constituée d'au moins 70 %, de préférence de 90 % d'IgA monomère sûre envers les virus ; comprenant les étapes ci-après :
(a) épuration d'une fraction contenant de l'immunoglobuline, au moyen d'une chromatographie à perméation sur gel et/ou d'une chromatographie thiophile, de manière à obtenir une IgA ayant une proportion de IgG inférieure à 10 % en poids sur l'immunoglobuline globale.
(b) exécution d'un procédé d'inactivation des virus.

9. Procédé selon la revendication 8, **caractérisé en ce que** le procédé d'inactivation des virus comprend un chauffage de l'IgA monomère.

10. Procédé selon l'une des revendications 8 ou 9, **caractérisée en ce qu'**on utilise, comme fraction contenant de l'immunoglobuline, du sérum sanguin humain, du plasma, des fractions de plasma ou du colostrum.

11. Procédé selon l'une des revendications 8 à 10, **caractérisé en ce qu'**on utilise comme matériau de départ la fraction Cohn II + III.

12. Procédé selon les revendications 8 à 11, **caractérisé en ce qu'**on soumet le matériau de départ à une pré-épuration.

13. Procédé selon la revendication 8, **caractérisé en ce qu'**on effectue une chromatographie à perméation sur gel, avec des effets mutuels, hydrophiles/hydrophobes ou électrostatiques.

14. Procédé selon l'une des revendications 8 à 13, **caractérisé en ce qu'**on effectue comme épuration supplémentaire une chromatographie à affinités.

15. Procédé selon l'une des revendications 8 à 14, **caractérisé en ce qu'**on effectue, en tant que procédé d'inactivation des virus, un traitement à la chaleur conduit à une température comprise et 40°C et 80°C, le mieux préféré entre 50°C et 65°C, sur une durée suffisante pour obtenir l'inactivation de virus.

16. Procédé selon l'une des revendications 8 à 15, **caractérisé en ce qu'**on effectue une dialyse relative à l'eau avant d'effectuer le traitement à la chaleur.

17. Procédé selon la revendication 8, **caractérisé en ce qu'**on utilise comme fraction contenant de l'immunoglobuline du plasma, ou une fraction de plasma, et Dépuration est effectuée sur une matrice thiophile.
